(19) Europäisches Patentamt
European Patent Office
Office européen des brevets

(11) Veröffentlichungsnummer: **0 275 436**
**B1**

(12) **EUROPÄISCHE PATENTSCHRIFT**

(45) Veröffentlichungstag der Patentschrift: **27.06.90**

(51) Int. Cl.$^5$: **C07C 65/34**, C07C 51/373, C07C 45/46

(21) Anmeldenummer: **87117865.3**

(22) Anmeldetag: **03.12.87**

(54) **Verfahren zur Herstellung von tert.-Amylbenzoylbenzosäure.**

(30) Priorität: **06.12.86 DE 3641793**

(43) Veröffentlichungstag der Anmeldung: **27.07.88 Patentblatt 88/30**

(45) Bekanntmachung des Hinweises auf die Patenterteilung: **27.06.90 Patentblatt 90/26**

(84) Benannte Vertragsstaaten: **AT BE CH DE ES FR GB IT LI NL**

(56) Entgegenhaltungen: **EP-A- 0 178 184**

(73) Patentinhaber: **BASF Aktiengesellschaft, Carl-Bosch-Strasse 38, D-6700 Ludwigshafen(DE)**

(72) Erfinder: **Henkelmann, Jochem, Dr., Ludwigshafener Strasse 44, D-6704 Mutterstadt(DE)**
Erfinder: **Eggersdorfer, Manfred Dr., Hannongstrasse 18, D-6710 Frankenthal(DE)**
Erfinder: **Grosch, Walter, Dr., Starenweg 1, D-6803 Edingen-Neckarhausen(DE)**
Erfinder: **Schuhmacher, Alfred, Dr., Von-Weber-Strasse 36, D-6700 Ludwigshafen(DE)**
Erfinder: **Siegel, Hardo, Dr., Hans-Purrmann-Allee 25, D-6720 Speyer(DE)**

EP 0 275 436 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von tert.-Amylbenzoylbenzoesäure durch Umsetzung von tert.-Amylbenzol mit Phthalsäureanhydrid in Gegenwart eines Friedel-Crafts-Katalysators.

Die Friedel-Crafts-Acylierung von Aromaten, d.h. die Einführung einer Acylgruppe in aromatische Verbindungen wie Alkylbenzol durch Einwirkung eines Acylierungsmittels auf Aromaten in Gegenwart bestimmter Metallhalogenide wie beispielsweise Aluminiumchlorid, ist allgemein bekannt, z.B. aus Houben-Weyl, Methoden der org. Chem. Bd. VII/2a, 1973, Seiten 15–39. Nachteilig an diesem Verfahren ist das Auftreten von Nebenreaktionen, insbesondere wenn alkylsubstituierte Aromaten acyliert werden. So kommt es zur Bildung harziger Nebenprodukte, sowie zur Abspaltung der Alkylgruppe und zu Isomerisierungen vor allem, wenn Aromaten mit sekundären oder tertiären Alkylresten umgesetzt werden. Wie aus verschiedenen Veröffentlichungen hervorgeht, sollen diese Nebenreaktionen durch ein Zusammenwirken von $AlCl_3$ und Chlorwasserstoff, der, sofern er nicht im Katalysator gegenwärtig ist, während der Friedel-Crafts-Acylierung entsteht (s. C.A. Olah, Friedel-Crafts and Related Reactions, Bd. I, Seite 207 und Bd. III, Teil I, S. 550 ff, Intersience 1964), verursacht werden. Um die Isomerisierung zurückzudrängen, werden als mögliche Maßnahmen das Entfernen der Halogenwasserstoffsäure durch Anlegen von vermindertem Druck am Reaktionssystem oder Durchleiten von trockener Luft oder Inertgasen empfohlen (s. Olah, Bd. III, S. 549 und dort zitierte Literatur). In der DE-AS 2 720 294 werden die erwähnten Maßnahmen aufgegriffen. Bei diesen Maßnahmen werden z.T. erhebliche Mengen der Reaktanden aus dem Reaktionsgemisch entfernt, was in der Folge neben einer aufwendigen Abgaswäsche zu schlechteren Ausbeuten führt. Auch sind diese Maßnahmen in der Technik nur schwer zu realisieren. So bereitet u.a. das Einbringen der benötigten Gasmengen in die Reaktionsmischung erhebliche Schwierigkeiten. Eine vollständige Unterdrückung der Alkylgruppenabspaltung und -isomerisierung ist mit diesen Maßnahmen nicht möglich, weshalb Produktgemische erhalten werden.

Besonders nachteilig wirkt sich die Isomerisierung der Alkylgruppe im Falle der Synthese von Amylbenzoylbenzoesäure aus, aus der durch Cyclisierung Amylanthrachinon hergestellt werden kann, das für die Herstellung von Wasserstoffperoxid von Bedeutung ist. Gemäß der DE-OS 2 013 299 werden die besten Ausbeuten an Wasserstoffperoxid pro kg Reaktionslösung erzielt, wenn ausschließlich 2-tert.-Amylanthrachinon und nicht das isomere 2-sek.-Isoamylanthrachinon verwendet wird. Nach den obengenannten Bedingungen werden aber stets Isomerengemische mit einem hohen Anteil an 4.-sek.-Isoamylbenzoylbenzoesäure, das nach Cyclisierung ebenfalls ein Gemisch aus 2-tert.-Amyl- und 2-sek.-Isoamylanthrachinon ergibt, erhalten.

EP-A 0 178 184 lehrt ein Verfahren zur Herstellung p-substituierter aromatischer Carbonylverbindungen durch die Lewis-Säure katalysierte Acylierung der entsprechend substituierten aromatischen Ausgangsverbindungen. Zur Erzielung einer hohen para-Selektivität werden die als Acylierungskatylsatoren verwendeten Lewis-Säuren durch den Zusatz einer Lewis-Base modifziert. Als geeignete Lewis-Basen werden in der Beschreibung eine Vielzahl anorganischer und organischer Verbindungen, unter anderem auch tertiäre Amine, genannt; in den Beispielen werden jedoch nur die Lewis-Basen Dimethylformamid, Butyronitril und insbesondere Lithiumchlorid benutzt. Eine Lehre zur Vermeidung der Lewis-Säure-katalysierten Gerüstumlagerung der Alkylgruppen höherer Alkylbenzole im Zuge der Acylierungsreaktion, insbesondere der Umlagerung der tert. Amylgruppe in die Isoamylgruppe, wird in dieser Schrift nicht gegeben.

Der Erfindung lag daher die Aufgabe zugrunde, ein verbessertes Verfahren zur Herstellung von 4-tert.-Amylbenzoylbenzoesäure zu finden, das deren Herstellung ohne oder mit nur einem geringen Anteil an 4-sek.-Isoamylbenzoylbenzoesäure erlaubt.

Demgemäß wurde ein Verfahren zur Herstellung von tert.-Amylbenzoylbenzoesäure durch Umsetzung von tert.-Amylbenzol mit Phthalsäureanhydrid in Gegenwart eines Friedel-Crafts-Katalysators gefunden, das dadurch gekennzeichnet ist, daß man die Umsetzung in Gegenwart eines tert. Amins oder Diamins durchführt.

Bei Verwendung von Phthalsäureanhydrid als Acylierungsmittel und Aluminiumchlorid als Friedel-Crafts-Katalysator läßt sich das Verfahren durch folgende Reaktionsgleichung beschreiben:

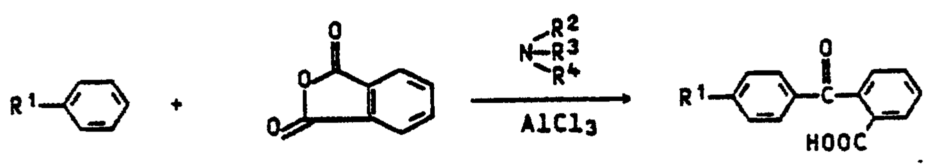


$R^1$ = Alkyl
$R^2$ bis $R^4$ = aliphatischer, cycloaliphatischer, araliphatischer, aromatischer Rest.

Das der Reaktionsmischung zugesetzte tertiäre Amin bindet den bei der Reaktion entstehenden Halogenwasserstoff in situ in Form des Ammoniumsalzes. Je nach Menge des tertiären Amins kann die Abspaltung und Isomerisierung der Alkylgruppe $R^1$ zurückgedrängt oder verhindert werden. Das Amin

kann aliphatisch, cycloalophatisch und/oder aromatisch substituiert sein, wobei auch zwei der Reste zu einem cyclischen, gegebenenfalls aromatischen System verknüpft sein können oder alle drei Reste ein bicyclisches System bilden können. Weiterhin können entsprechend substituierte Diamine anstelle der Monoamine verwendet werden.

Aliphatische Reste sind z.B. Alkyl- oder Alkenylreste, insbesondere mit 1 bis 20, vorzugsweise 1 bis 8 Kohlenstoffatomen, z.B. Methyl-, Ethyl-, Propyl-, Isopropyl-, n-Butyl-, iso-Butyl-, tert.-Butyl-, Pentyl-, Isopentyl-, Hexyl-, Isooctyl-, Nonyl- oder Dodecylreste sowie iso-Butenyl-, n-Pentenyl-, n-Hexenyl- oder Octenylreste. Cycloaliphatische Reste sind Cycloalkyl- oder Cycloalkenylreste mit 3 bis 8, insbesondere 5 und 6 Ringgliedern, die noch ein weiteres Heteroatom wie Stickstoff oder Sauerstoff enthalten können, z.B. Cyclopropyl-, Cyclobutyl-, Cyclopentyl-, Cyclopentenyl, Cyclohexyl-, Cyclohexenyl-, Cyclooctyl- oder Furfurylreste. Araliphatische Reste sind z.B. Aralkylreste oder Alkylarylreste mit 7 bis 12 Kohlenstoffatomen, z.B. der Benzyl- oder Phenylethylrest. Aromatische Reste sind z.B. der Phenyl-, Naphthyl- oder 4-Pyridylrest.

Zwei der Reste $R^2$ bis $R^4$ können auch miteinander zu einem cyclischen System, z.B. zu einem Pyrrolidin-, Piperidin-, Piperazin-, Pyridin-, Chinolin-, Chinoxalin-, Acridin- oder Pyrrolsystem verknüpft sein.

Weiterhin können alle 3 Reste miteinander ein bicyclisches System, z.B. ein 1-Aza- oder 1,4-Diazabicyclo[2,2,2]octan oder 1-Azabicyclo[2,2,1]-heptan, bilden.

Die genannten Reste können noch unter den Reaktionsbedingungen inerte Substituenten wie $C_1$- bis $C_4$-Alkylgruppen tragen.

Beispielsweise seien folgende tertiäre Amine genannt: Trimethyl-, Triethyl-, Tri-n-propyl-, Triisopropyl-, Tri-n-butyl-, Triisobutyl-, Tri-amyl-, Trihexyl-, Dimethylethyl-, Diisopropylethyl-, Diethylisopropyl-, Dimethyl-tert.-butyl-, Dimethyldodecyl-, N,N-Dimethylcyclohexyl-, Dicyclohexylethyl-, N,N-Dimethylenzyl-, N,N-Dietyhl-α-naphthyl-, N-Methyl-N-phenyl-1-naphthyl- oder Trifurfurylamin sowie N,N-Diethylanilin, N-Methylpyrrolidin, N-Ethylpiperidin, N-Propylpiperidin, Pyridin, α,β- oder γ-Picolin, 2,4-oder 2,6-Lutidin, N-Ethylpyrrol, N-Methylimidazol, Chinolin, Isochinolin, Acridin, p-Dimethylaminopyridin, N-4(4-Pyridyl)-pyridin, N,N'-Diethylpiperazin, N,N,N',N'-Tetraethyl-1,3-propandiamin und 1,4-Diazabicyclo[2,2,2]octan.

Besonders vorteilhaft können tertiäre alkyl- und/oder cycloalkylsubstituierte Amine sowie cyclische oder bicyclische Amine verwendet werden.

Als Friedel-Crafts-Katalysatoren können die an sich üblichen Verbindungen wie Eisen(III)-chlorid, Bortrifluorid, Titantetrachlorid, Aluminium(III)-bromid und Aluminium(III)-Chlorid verwendet werden, wobei die genannten Aluminiumhalogenide bevorzugt sind.

Die Menge an tertiärem Amin liegt im allgemeinen bei 0,1 bis etwa 1,5, vorzugsweise 0,1 bis 1 mol je Äquivalent Carbonsäureanhydrid. Die Aluminiumhalogenidmenge beträgt zweckmäßig etwa 2 bis 3,5 mol, insbesondere 2,1 bis 2,5 mol je Äquivalent Säureanhydrid.

Die Aktivität des Friedel-Crafts-Katalysators wird, je nach verwendetem Amin, unterschiedlich stark modifiziert, weshalb die eingesetzte Aluminiumhalogenidmenge stark von der Natur des Amins abhängt, wobei sterisch gehinderte aliphatische Amine wie Triethylamin, Diisopropylethylamin und Dicyclohexylethylamin oder bicyclische Amine wie 1,4-Diazabicyclo[2,2,2]octan besonders vorteilhaft sind. Die jeweils optimale Menge Katalysator kann in Vorversuchen leicht ermittelt werden.

Als Acylierungsmittel wird Phthalsäureanhydrid verwendet.

Die Reaktion kann ohne oder vorteilhaft in Gegenwart eines Lösungsmittels ausgeführt werden, wobei als Lösungsmittel die konventionellen Lösungsmittel für Friedel-Crafts-Reaktionen in Betracht kommen, z.B. Chlorbenzol, Dichlorbenzol, 1,2-Dichlorethan, Trichlorethylen, 1,2-Dichlorpropan, Schwefelkohlenstoff, Nitromethan oder Nitrobenzol. Die Lösungsmittelmenge ist nicht kritisch, im allgemeinen können bis 1000 g je Mol Alkylbenzol verwendet werden.

Die Durchführung der Reaktion kann in an sich bekannter Weise erfolgen, wobei man die Ausgangsstoffe bei Temperaturen von –20 bis 100, vorzugsweise 0 bs 60, insbesondere 10 bis 40°C und bei erhöhtem oder vermindertem Druck, vorzugsweise bei Normaldruck umsetzt.

Zweckmäßigerweise wird das Phthalsäureanhydrid zusammen mit dem Lösungsmittel vorgelegt, der Friedel-Crafts-Katalysator in Portionen zugegeben und der Alkylaromat im Gemisch mit dem Amin zugetropft. Im allgemeinen werden stöchiometrische Mengen an Säureanhydrid und Alkylaromat verwendet, es ist aber auch möglich, einen der beiden Ausgangsstoffe im Überschuß einzusetzen oder z.B. das Amylbenzol aus Lösungsmittel zu verwenden.

Nach beendeter Reaktion erfolgt die Aufarbeitung und Isolierung der Produkte in an sich bekannter Weise, z.B. in dem man die entstandenen Katalysatorkomplexe mit Wasser zersetzt und das gewünschte Keton durch Extraktion oder Kristallisation gewinnt.

Nach dem erfindungsgemäßen Verfahren ist es überraschend möglich, alkylsubstituiertes, insbesondere tert.-alkylsubstituiertes Benzol nach Friedel-Crafts zu acylieren, ohne daß eine starke Isomerisierung des Alkylrestes auftritt, was insbesondere im Fall der Synthese von tert.-Amylbenzoylbenzoesäure von Interesse ist. Durch die Menge an tertiärem Amin kann das Verhältnis von tertiär- zu sekundär-Alkyl gezielt gesteuert werden. Die nach dem beschriebenen Verfahren herstellbaren aromatischen Ketone sind wertvolle Zwischenprodukte z.B. für Farbstoffe, Hilfsstoffe, Pflanzenschutzmittel und Pharmaka. Tert.-Amylbenzoylbenzoesäure ist ein wichtiges Zwischenprodukt zur Herstellung von tert.-Amylanthrachinon, das für die Produktion von Wasserstoffperoxid benötigt wird.

Beispiele 1 bis 3

Herstellung von 4-tert.-Amyl- und 4-sek.-Isoamylbenzoylbenzoesäure

55 g (0,37 mol) Phthalsäureanhydrid wurden zusammen mit Aluminiumchlorid in 260 g o-Dichlorbenzol vorgelegt und eine Lösung aus 55 g (0,37 mol) tert.-Amylbenzol und dem in der nachfolgenden Tabelle aufgeführten tertiären Amin innerhalb von 5 Stunden zugetropft. Die Reaktionstemperatur wurde während dieser Zeit zwischen 17 und 20°C gehalten. Anschließend wurde 1 Stunde bei 40°C nachgerührt.

Zur Aufarbeitung wurde die Reaktionsmischung in verdünnte Schwefelsäure gegossen und die organische Phase mit verdünnter Natronlauge extrahiert. Die Amylbenzoylbenzoesäure wurde aus der wäßrigen Phase mittels Schwefelsäure ausgefällt und anschließend abfiltriert und getrocknet. Die Ausbeute und Zusammensetzung der so erhaltenen Isomerengemische sind Tabelle 1 zu entnehmen.

Tabelle 1

Umsetzung von tert.-Amylbenzol mit Phthalsäureanhydrid in Gegenwart von $AlCl_3$ und tertiärem Amin

| Beispiel | Amin | Menge g | Amin mol | Menge g | $AlCl_3$ mol | Ausbeute g | % | Isomerenverhältnis tert/sek |
|---|---|---|---|---|---|---|---|---|
| 1 | 1,4-Diazabicyclo[2,2,2]octan | 21,9 | 0,19 | 105 | 0,78 | 82 | 75 | 86/14 |
| 2 | Diisopropylethylamin | 26 | 0,2 | 105 | 0,78 | 93 | 85 | 70/30 |
| 3 | Triethylamin | 40 | 0,4 | 158 | 1,18 | 71 | 65 | 99,5/0,5 |

## Patentansprüche

1. Verfahren zur Herstellung von tert.-Amylbenzoylbenzoesäure durch Umsetzung von tert.-Amylbenzol mit Phthalsäureanhydrid in Gegenwart eines Friedel-Crafts-Katalysators, dadurch gekennzeichnet, daß man die Umsetzung in Gegenwart eines tertiären Amins oder eines tertiären Diamins durchführt.
2. Verfahren nach Anspruch 1, dadurch gekennzeichnet, daß man ein tert. alkyl- und/oder cycloalkylsubstituiertes Amin verwendet.
3. Verfahren nach Anspruch 1, dadurch gekennzeichnet, daß man ein cyclisches oder bicyclisches tert.-Amin verwendet.
4. Verfahren nach Anspruch 1, dadurch gekennzeichnet, daß man 0,1 bis 1,5 mol tert.-Amin je Äquivalent Phthalsäureanhydrid verwendet.
5. Verfahren nach Anspruch 1, dadurch gekennzeichnet, daß man als Friedel-Crafts-Katalysator Aluminiumhalogenide verwendet.

## Claims

1. A process for preparing tert-amylbenzoylbenzoic acid by reacting tert-amylbenzene with phthalic anhydride in the presence of a Friedel-Crafts catalyst, which comprises performing the reaction in the presence of a tertiary amine or diamine.
2. A process as claimed in claim 1, wherein a tertalkyl- or cycloalkyl-substituted amine is used.
3. A process as claimed in claim 1, wherein a cyclic or bicyclic tertiary amine is used.
4. A process as claimed in claim 1, wherein from 0.1 to 1.5 moles of tertiary amine are used per equivalent of phthalic anhydride.
5. A process as claimed in claim 1, wherein the Friedel-Crafts catalyst used is an aluminum halide.

## Revendications

1. Procédé de préparation d'acide tert.-amylbenzoylbenzoïque par réaction de tert.-amylbenzène avec l'anhydride phtalique en présence d'un catalyseur de Friedel-Crafts, caractérisé en ce qu'on conduit la réaction en présence d'une amine tertiaire ou d'une diamine tertiaire.
2. Procédé selon la revendication 1, caractérisé en ce qu'on utilise une amine tertiaire alkyl- et/ou cycloalkyl-substituée.
3. Procédé selon la revendication 1, caractérisé en ce qu'on utilise une amine tertiaire cyclique ou bicyclique.
4. Procédé selon la revendication 1, caractérisé en ce qu'on utilise 0,1 à 1,5 mole d'amine tertiaire par équivalent d'anhydride phtalique.
5. Procédé selon la revendication 1, caractérisé en ce qu'on utilise des halogénures d'aluminium comme catalyseurs de Friedel-Crafts.